(19)

Europäisches Patentamt

European Patent Office

Office européen des brevets

(11) **EP 3 584 577 A1**

(12) **EUROPEAN PATENT APPLICATION**
published in accordance with Art. 153(4) EPC

(43) Date of publication:
**25.12.2019 Bulletin 2019/52**

(51) Int Cl.:
***G01N 33/543*** (2006.01)      ***G01N 33/483*** (2006.01)

(21) Application number: **18754094.3**

(22) Date of filing: **16.01.2018**

(86) International application number:
**PCT/JP2018/001060**

(87) International publication number:
**WO 2018/150787 (23.08.2018 Gazette 2018/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **14.02.2017 JP 2017025362**

(71) Applicant: **Fujifilm Corporation**
**Minato-ku**
**Tokyo 106-8620 (JP)**

(72) Inventor: **SATO Keiichiro**
**Ashigarakami-gun**
**Kanagawa 258-8538 (JP)**

(74) Representative: **Klunker IP**
**Patentanwälte PartG mbB**
**Destouchesstraße 68**
**80796 München (DE)**

(54) **IMMUNOLOGICAL TEST DEVICE**

(57) An immunological examination apparatus 10 includes an imaging unit 12 that images a chromatographic test piece 1; a determination unit 25 that determines presence or absence of dropping of the sample solution onto the chromatographic test piece 1 on the basis of an image acquired by the imaging unit 12; and a notification unit 13 that gives a notification of a determination result of the determination unit 25. The determination unit 25 acquires a brightness change rate of the chromatographic test piece 1 in a longitudinal direction x in a determination region R set in the image, and determines that the sample solution is dropped in a case where a brightness difference $\Delta$ in which an absolute value of the brightness change rate is equal to or greater than a first threshold value $TV_1$ is present.

FIG. 3

10

EP 3 584 577 A1

Printed by Jouve, 75001 PARIS (FR)

**Description**

**BACKGROUND OF THE INVENTION**

1. Field of the Invention

The present invention relates to an immunological examination apparatus.

2. Description of the Related Art

Immunological examination apparatuses that detect or quantify antigens, such as viruses in a sample, are used for examination of viral infectious diseases, such as influenza, utilizing an antigen-antibody reaction. In this type of immunological examination apparatus, typically, a sample solution is dropped onto a chromatographic test piece. The sample solution dropped to the test piece flows through the test piece in a longitudinal direction, and passes through a reaction section provided in the test piece. The reaction section is colored by capturing the antigens in the sample solution by the antigen-antibody reaction, and this reaction section is imaged, and the antigens are detected or quantified on the basis of the coloration intensity of the reaction section in the acquired image.

The coloration of the reaction section is influenced by the time (reaction time) during which the reaction section is in contact with the sample solution, and even though the concentration of the antigens in the sample solution is the same, the coloration intensity of the reaction section is different in a case where the reaction time is different. In the examination apparatus described in JP2010-032447A, an addition state imaging sensor that images an addition section on which the sample solution is dropped in the test piece is further provided separately from an imaging sensor that images the reaction section from a viewpoint of managing the reaction time with high accuracy. Time measurement is started simultaneously when being detected that an appropriate amount of the sample solution has been dropped onto the test piece, on the basis of an image acquired by the addition state imaging sensor, the reaction section is imaged after lapse of a predetermined time from the start of the measurement, and the reaction time is managed.

**SUMMARY OF THE INVENTION**

Since the sample solution is dropped onto the test piece by an operator, dropping forgetting may occur. For example, the above reaction time is typically set to about 10 minutes. However, in a case where analysis proceeds with the sample solution being not dropped, the time required for the analysis including this reaction time is wasted. From a viewpoint of an increase in efficiency of analysis work, it is desirable to early detect the dropping forgetting and notify the operator of the dropping forgetting.

In the examination apparatus described in JP2010-032447A, the intensity of an image of the addition section acquired by the addition state imaging sensor is monitored, the intensity in a state where the sample solution is not dropped is used as a reference value, and whether or not the sample solution has reached an appropriate amount is detected on the basis of a difference from the reference value. In the above detection operation, the state where the sample solution is not dropped, which serves as the reference value, that is, the dropping forgetting, cannot be detected.

Additionally, in the examination apparatus described in JP2010-032447A, in a case where the amount of the sample solution does not reach an appropriate amount within a predetermined time after the test piece is mounted on the examination apparatus, a display for prompting remeasurement is output to a display unit. Even in a case where the dropping forgetting is consequently recognized by the operator on the basis of this display, the additional addition state imaging sensor that images the addition section of the test piece is required separately from the imaging sensor that images the reaction section of the test piece. Thus, it is disadvantageous for reduction in size and cost reduction of an examination apparatus.

The invention has been made in view of the above-described circumstances, and an object thereof is to provide an immunological examination apparatus capable of giving an early notification of dropping forgetting of a sample solution onto a chromatographic test piece.

An immunological examination apparatus of an aspect of the invention is an immunological examination apparatus that images a chromatographic test piece onto which a sample solution is dropped and on which the sample solution dropped is made to flow in a longitudinal direction and that analyzes the sample solution on the basis of an acquired image. The immunological examination apparatus comprises an imaging unit that images the chromatographic test piece, a determination unit that determines presence or absence of a dropping of the sample solution onto the chromatographic test piece on the basis of an image acquired by the imaging unit, and a notification unit that gives a notification of a determination result of the determination unit. The determination unit acquires a brightness change rate of the chromatographic test piece in the longitudinal direction in a determination region set in the image, and determines that the sample solution has been dropped in a case where a brightness difference in which an absolute value of the brightness change rate is equal to or greater than a first threshold value is present.

Additionally, an immunological examination apparatus of an aspect of the invention is an immunological examination apparatus that images a chromatographic test piece onto which a sample solution is dropped and on which the sample solution dropped is made to flow in a longitudinal direction and that analyzes the sample solution on the basis of an acquired image. The immunological examination apparatus comprises an imaging unit that images the chromatographic test piece, a determination unit that determines presence or absence of a dropping of the sample solution onto the chromatographic test piece on the basis of an image acquired by the imaging unit, and a notification unit that gives a notification of a determination result of the determination unit. The determination unit detects a dark pixel of which a brightness value is equal to or smaller than a second threshold value, in a determination region set in the image, and determines that the sample solution has been dropped in a case where a predetermined number or more of the dark pixel is present.

An immunological examination apparatus of an aspect of the invention is an immunological examination apparatus that images a chromatographic test piece onto which a sample solution is dropped and on which the sample solution dropped is made to flow in a longitudinal direction and that analyzes the sample solution on the basis of an acquired image. The immunological examination apparatus comprises an imaging unit that images the chromatographic test piece, a determination unit that determines presence or absence of a dropping of the sample solution onto the chromatographic test piece on the basis of an image acquired by the imaging unit, and a notification unit that gives a notification of a determination result of the determination unit. The determination unit detects a bright pixel of which a brightness value is equal to or greater than a third threshold value for each of a plurality of images in a determination region set in the image, and determines that the sample solution has been dropped in a case where a pixel number of the bright pixel of each of the images decreases in order of acquisition of the images.

According to the invention, it is possible to provide the immunological examination apparatus capable of giving an early notification of dropping forgetting of the sample solution onto the chromatographic test piece.

## BRIEF DESCRIPTION OF THE DRAWINGS

Fig. 1 is a perspective view of an example of a cartridge that houses a chromatographic test piece for describing an embodiment of the invention.
Fig. 2 is a schematic view of the chromatographic test piece of Fig. 1.
Fig. 3 is a block view of an example of an immunological examination apparatus for describing the embodiment of the invention.
Fig. 4 is a schematic view of an imaging unit of the immunological examination apparatus of Fig. 3.
Fig. 5 is a schematic view illustrating an example of a color tone of the chromatographic test piece.
Fig. 6 is a schematic view illustrating another example of the color tone of the chromatographic test piece.
Fig. 7 is a graph illustrating an example of brightness information of an image of the chromatographic test piece.
Fig. 8 is a graph illustrating a brightness change rate acquired from the brightness information of Fig. 7.
Fig. 9 is a flowchart illustrating an example of determination processing to be executed by a control unit of the immunological examination apparatus of Fig. 3.
Fig. 10 is a schematic view illustrating another example of the color tone of the chromatographic test piece.
Fig. 11 is a graph illustrating another example of the brightness information of the image of the chromatographic test piece.
Fig. 12 is a flowchart illustrating another example of the determination processing to be executed by the control unit of the immunological examination apparatus of Fig. 3.
Fig. 13 is a flowchart illustrating another example of the determination processing to be executed by the control unit of the immunological examination apparatus of Fig. 3.
Fig. 14 is a flowchart illustrating another example of the determination processing to be executed by the control unit of the immunological examination apparatus of Fig. 3.

## DESCRIPTION OF THE PREFERRED EMBODIMENTS

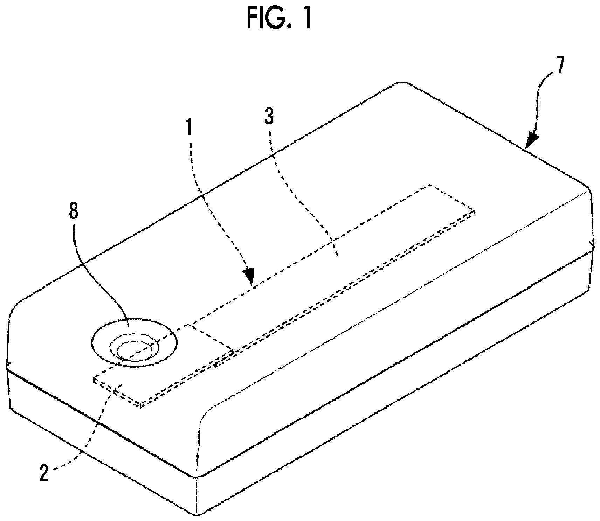

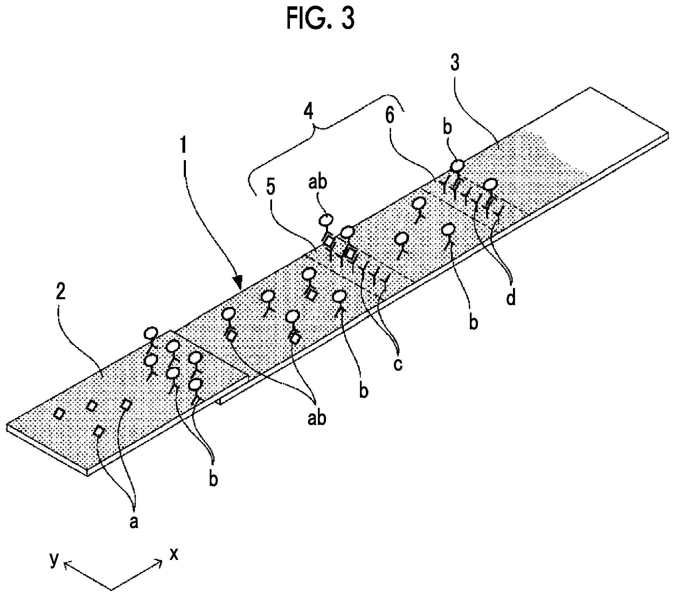

Figs. 1 and 2 illustrate an example of a chromatographic test piece to be used for analysis of a sample solution and a cartridge housing the chromatographic test piece, which are provided for describing an embodiment of the invention.

A chromatographic test piece (hereinafter referred to as a test piece) 1 illustrated in Figs. 1 and 2 is used for analysis in which antigens, such as viruses in the sample solution, are detected or quantified utilizing an antigen-antibody reaction. The test piece 1 is, for example, a beltlike thin piece made of porous materials, such as cellulose, and is typically white. The test piece 1 comprises a dropping section 2 provided at an end part on one side in a longitudinal direction x, and a spreading section 3 provided adjacent to the dropping section 2 in the longitudinal direction x. The sample solution is dropped onto the dropping section 2, the sample solution dropped onto the dropping section 2 flows from the dropping section 2 to the spreading section 3 by the capillary phenomenon, and further flows through the spreading section 3 in the longitudinal direction x toward an end part opposite to the dropping section 2.

The dropping section 2 is provided with labeled antibodies b labeled with golden colloidal particles. The labeled antibodies b are dissolved in the sample solution dropped onto the dropping section 2, and are bonded with antigens a to form antigen-antibody complexes ab in a case where the antigen a is included in the sample solution. The antigen-antibody complexes ab are moved riding on the flow of sample solution, and the surplus labeled antibodies b, which have remained without being bonded with the antigens a, are moved riding on the flow of the sample solution.

The spreading section 3 is provided with a reaction section 4. The reaction section 4 has a determination line 5 for detecting or quantifying the antigens a in the sample solution, and further has a control line 6 for detecting that the sample solution has appropriately flowed to the test piece 1 in the present example. The determination line 5 and the control line 6 are provided across the spreading section 3 in a lateral direction y orthogonal to the longitudinal direction x, and are the control line 6 is provided downstream of the flow of the sample solution than the determination line 5.

First capture antibodies c bonded with the antigens a are fixedly provided on the determination line 5 for detecting or quantifying the antigens a in the sample solution. The antigen-antibody complexes ab, which are moved riding on the flow of the sample solution, are captured by the first capture antibodies c of the determination line 5, and are fixed to the determination line 5. The determination line 5 is colored with the golden colloidal particles adhering to the labeled antibodies b of the antigen-antibody complexes ab by the antigen-antibody complexes ab being fixed to the determination line 5, and the coloration of the determination line 5 becomes stronger as the antigen-antibody complexes ab to be fixed become increase. The coloration of the determination line 5 is optically detected as changes in absorbance, and the antigens a in the sample solution are detected or quantified.

Second capture antibodies d bonded with the labeled antibodies b are fixedly provided in the control line 6 for detecting that the sample solution has appropriately flowed to the test piece 1. The surplus labeled antibodies b moved riding on the flow of the sample solution pass by the determination line 5 without being captured by the first capture antibodies c, are captured by the second capture antibodies d of the control line 6, and are fixed to the control line 6. The control line 6 is colored with the golden colloidal particles adhering to the labeled antibodies b as the labeled antibodies b being fixed to the control line 6, and the coloration of the control line 6 becomes stronger as the labeled antibodies b increases to be fixed. That is, the control line 6 is colored by coming into contact with the sample solution irrespective of whether the antigens a are included in the sample solution. The coloration of the control line 6 is optically detected as changes in absorbance, and is detected that the sample solution has appropriately flowed to the test piece 1.

The test piece 1 is housed in a cartridge 7 and used. The cartridge 7 is provided with an opening 8 that faces the dropping section 2 of the housed test piece 1, and the sample solution is dropped onto the dropping section 2 through the opening 8. The cartridge 7 is made of transparent resin materials, and the coloration of the reaction section 4 (the determination line 5 and the control line 6) of the test piece 1 is optically detected through the cartridge 7.

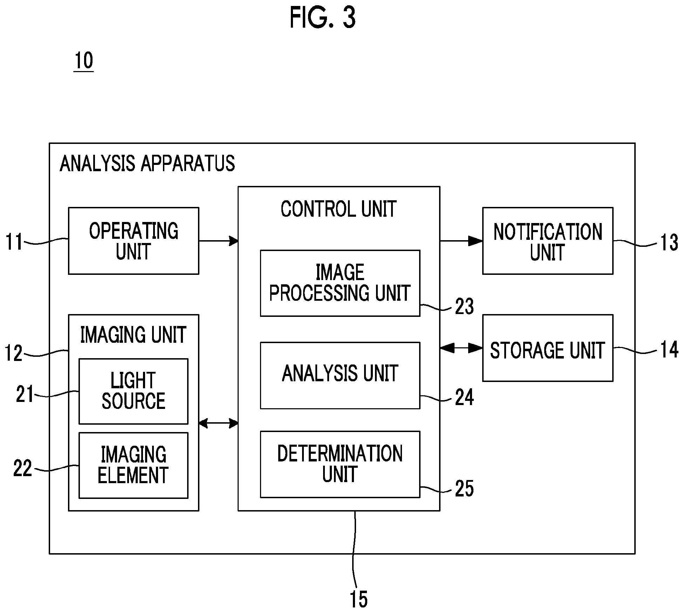

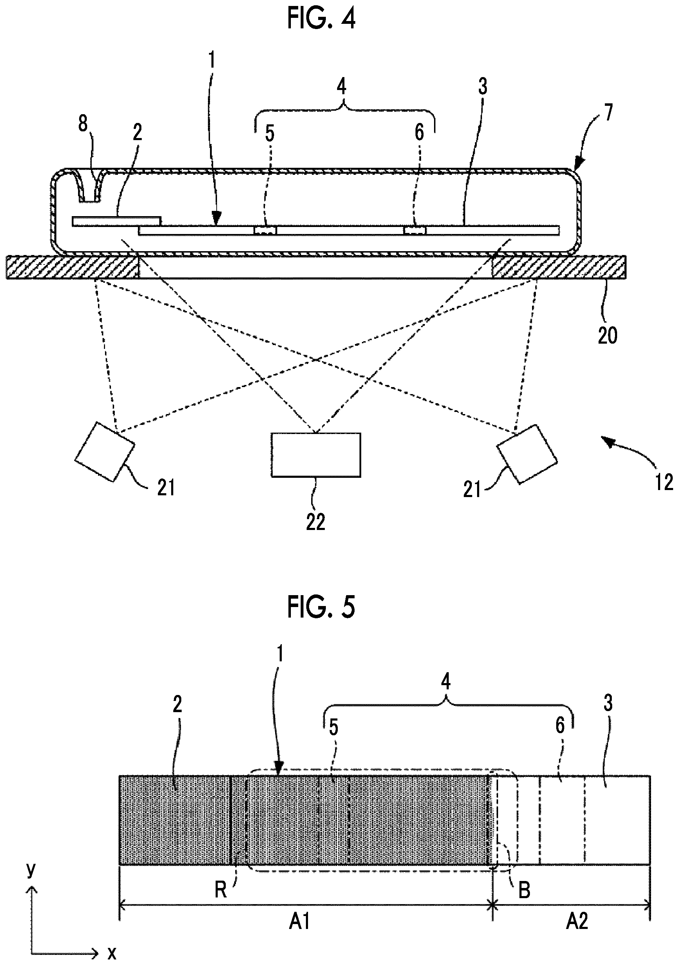

Figs. 3 and 4 illustrate an example of the immunological examination apparatus that analyzes using the test piece 1.

The immunological examination apparatus 10 comprises an operating unit 11, an imaging unit 12, a notification unit 13, a storage unit 14, and a control unit 15 that controls the operation of the operating unit 11, the imaging unit 12, the notification unit 13, and the storage unit 14.

The operating unit 11 receives various instructions (for example, an analysis start instruction and the like) of an operator. The operating unit 11 is constituted of, for example, hardware keys, such as switches. The instructions received by the operating unit 11 are input to the control unit 15.

The imaging unit 12 optically detects the coloration of the reaction section 4 (the determination line 5 and the control line 6) of the test piece 1. The imaging unit 12 includes an installation unit 20 in which the cartridge 7 housing the test piece 1 is installed, a light source 21, such as a light emitting diode (LED), and an imaging element 22, such as a charge-coupled device (CCD) or a complementary metal oxide semiconductor (CMOS). The test piece 1 of the cartridge 7 installed in the installation unit 20 is illuminated by the light source 21, and is imaged by the imaging element 22 in the illuminated state. The acquired image of the test piece 1 is input to the control unit 15.

The notification unit 13 notifies the operator of various kinds of information (for example, analysis results and the like). The notification unit 13 may include, for example, display panels, such as a liquid crystal display (LCD) and an organic electro-luminescence display (OELD), and may perform the notification of the information by displaying images and texts on display screens of the display panels. Additionally, the notification unit 13 may include indicating lamps, such as a light emitting diode (LED), and may perform the notification of the information by lighting, blinking, and the like of the indicating lamps. Additionally, the notification unit 13 may include buzzers, and may perform the notification of the information by voice.

The storage unit 14 stores control programs and control data to be executed by the control unit 15, and stores various kinds of information, such as the analysis results. The storage unit 14 is constituted of, for example, storage media, such as a flash memory, a hard disk, a read-only memory (ROM), and a random access memory (RAM).

The control unit 15 controls the operation of the operating unit 11, the imaging unit 12, the notification unit 13, and the storage unit 14 by operating in accordance with the control programs. Additionally, the control unit 15 also functions as an image processing unit 23 and an analysis unit 24 by operating in accordance with the control programs.

The image processing unit 23 creates brightness information obtained by digitizing the coloration intensity of the reaction section 4 (the determination line 5 and the control line 6) appearing on the image of the test piece 1 depending on the brightness of the image.

The analysis unit 24 detects or quantifies the antigens a in the sample solution on the basis of the coloration intensity of the determination line 5 indicated depending on the brightness information. Additionally, the analysis unit 24 detects that the sample solution has appropriately flowed to the test piece 1 on the basis of the coloration intensity of the control line 6 indicated depending on the brightness information. Then, the analysis unit 24 creates the above analysis results.

In the above configuration, the control unit 15 executes a predetermined analysis process in a case where the analysis start instruction is input from the operating unit 11 by operating in accordance with the control programs. The predetermined analysis process includes a step of counting lapse of a predetermined reaction time from the start of analysis, a step of making the imaging unit 12 image the test piece 1 after the lapse of the predetermined reaction time, a step of creating the brightness information obtained by digitizing the coloration intensity of the reaction section 4 (the determination line 5 and the control line 6) appearing on the acquired image of the test piece 1 depending on the brightness of the image, and a step of creating analysis results on the basis of the created brightness information. In addition, the reaction time is the time during which the reaction section 4 is in contact with the sample solution, and the predetermined reaction time is the time during which the reaction section 4 is colored with sufficient intensity for detection in a case where the sample solution including antigens in a predetermined concentration has flowed through the horizontally-placed test piece 1, and is appropriately set.

Then, the control unit 15 notifies the notification unit 13 of the analysis results created through the above predetermined analysis process, and stores the analysis results in the storage unit 14.

Moreover, the control unit 15 functions also as a determination unit 25 by operating in accordance with the control programs, determines whether or not the sample solution is dropped onto the test piece 1, and executes the processing of notifying the notification unit 13 of determination results. The determination processing will be described below.

In the present example, it is detected that the sample solution has appropriately flowed to the test piece 1 on the basis of the coloration intensity of the control line 6, and whether or not the sample solution has been dropped into the test piece 1 can be determined also by this detection result. However, until this detection result is acquired, it is required that the above predetermined reaction time lapses and an image for creating the brightness information obtained by digitizing the coloration intensity of the reaction section 4, that is, an analyzing image used for the analysis of the sample solution is acquired. From a viewpoint of early performing notification of dropping forgetting of the sample solution onto the test piece 1, the execution of the above determination processing and the notification of the determination results are performed at least at a timing before the acquisition timing of the analyzing image.

The hardware structure of the control unit 15 that performs various kinds of processing as the image processing unit 23, the analysis unit 24, and the determination unit 25 includes a central processing unit (CPU) that is a general-purpose processor, a programmable logic device (PLD) that is a processor capable of changing a circuit configuration after manufacture of a field programmable gate array (FPGA) or the like, and exclusive electric circuits, which are processors having circuit configurations exclusively designed to execute specific processing, such as an application specific integrated circuit (ASIC).

The respective processing units (the image processing unit 23, the analysis unit 24, and the determination unit 25, and the like) of the control unit 15 may be constituted of one of above various processors for each processing unit, or may be constituted of a combination of two or more same or different types of processors (for example, a combination of a plurality of the FPGAs or a combination of the CPU and the FPGA). Additionally, the plurality of processing units may be constituted of one processor.

As an example in which the plurality of processing units are constituted of the one processor, firstly, as represented by a computer, such as a client or a server, there is a form in which one processor is constituted of a combination of one or more CPUs and software and this processor functions as the plurality of processing units. Secondly, as represented by a system-on-chip (SOC) or the like, there is a form in which a processor, which realizes functions of an overall system including the plurality of processing units with one integrated circuit (IC) chip, is used. In this way, the various processing units are configured by using one or more of the above various processors as the hardware structure(s). Moreover, the hardware structures of these various processors are more specifically circuitries in which circuit elements, such as semiconductor elements, are combined together.

Hereinafter, a method of determining whether or not the sample solution is dropped onto the test piece 1 will be described.

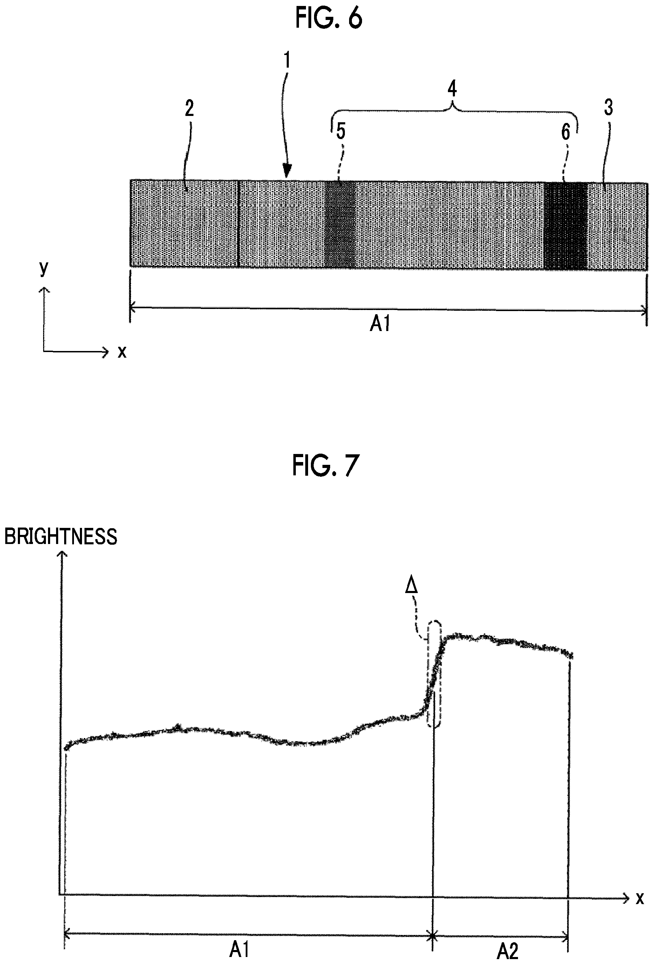

Fig. 5 is a schematic view illustrating a color tone of the test piece 1 while the sample solution flows, and Fig. 6 is a schematic view illustrating the color tone of the test piece 1 after the sample solution had flowed.

The test piece 1 is white in a state where the sample solution is not dropped, and a region that is wetted in the sample solution becomes dark as the sample solution is dropped and wetted. As illustrated in Fig. 5, in the test piece 1 while the sample solution flows, that is, the test piece 1 before a leading end of the flow of the sample solution arrives at the end part of the test piece 1 opposite to the dropping section 2, a boundary B of the color tone is formed between an already-spread region A1 that is already wetted in the sample solution, and a non-spread region A2. In the image of the test piece 1, the boundary B of this color tone appears as a brightness difference extending in the lateral direction y of the test piece 1.

Thus, by detecting the brightness difference of the image of the test piece 1, it is possible to determine whether or not the sample solution is dropped onto the test piece 1. That is, it can be determined that the sample solution is dropped in a case where the brightness difference has been detected, it can be determined that the sample solution is not dropped in a case where the brightness difference is not detected.

However, as illustrated in Fig. 6, in the test piece 1 through which the sample solution has flowed, that is, the test piece 1 in which the leading end of the flow of the sample solution has arrived at the end part of the test piece 1 opposite to the dropping section 2, the entire test piece 1 becomes the already-spread region A1, and the boundary B disappears. Accordingly, an image (determination image) of the test piece 1 used for determining whether or not the sample solution is dropped is acquired at least before the sample solution dropped onto the test piece 1 has flowed through the test piece 1.

Although the time required until the sample solution has flowed through the test piece 1 after being dropped onto the test piece 1 is based also on the length of the test piece 1 and the inclination of the longitudinal direction x (the flow direction of the sample solution) of the test piece 1 with respect to the horizontal, the time is typically about 2 minutes. From this, the determination image can be, for example, an image acquired after lapse of 30 seconds to 1 minute and 30 seconds after the sample solution is dropped onto the test piece 1.

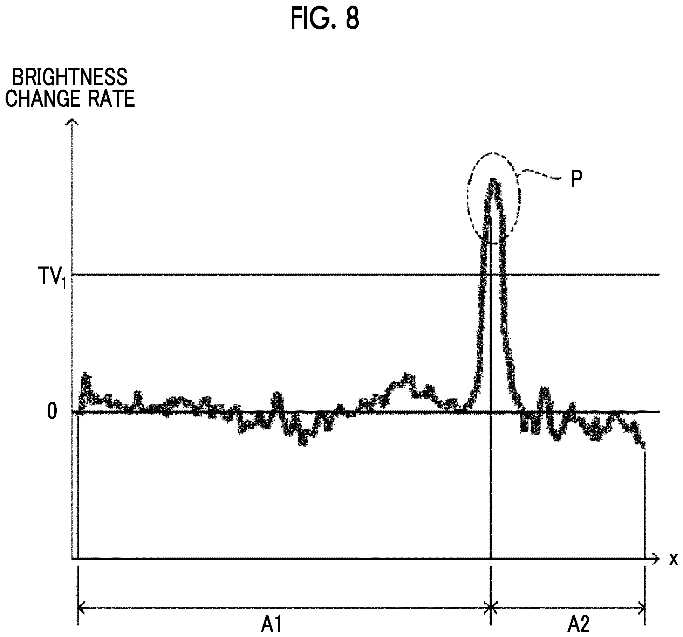

Fig. 7 illustrates an example of brightness information of the determination image, and Fig. 8 illustrates a brightness change rate acquired from the brightness information of Fig. 7.

The brightness information illustrated in Fig. 7 is obtained by digitizing the image of the test piece 1 while the sample solution illustrated in Fig. 5 flows, depending on the brightness, and illustrates changes in brightness of the test piece 1 in the longitudinal direction x. The boundary B of the color tone formed between the already-spread region A1 and the non-spread region A2 of the test piece 1 appears as a brightness difference $\Delta$ in the brightness information.

A technique for the digitization is not particularly limited. However, in a case where image signals of a YUV (a difference between Y: a brightness signal or U: a brightness signal and a blue component or a difference between V: a brightness signal and a red component) format or a YCbCr (a difference between Y: a brightness signal or Cb: a brightness signal and the blue component or a difference between Cr: a brightness signal and the red component) format are output from the imaging unit 12, there is a technique directly using the Y signal. This method is simple because the signals output from the imaging unit 12 can be directly used and has excellent versatility because the method does not depend on the color of the sample solution.

Additionally, any one signal out of a U signal and a V signal of the YUV format or any one signal out of a Cb signal and a Cr signal of the YCbCr format can also be used instead of the Y signal. Additionally, in a case where image signals of RGB (R: red component, G: green component, and B: blue component) format or Raw (Raw image format) format are output from the imaging unit 12, any one signal out of the R signal, the G signal, and the B signal of the RGB format or any one signal out of the R signal, the G signal, and the B signal of the of Raw format can also be used. In this method, a signal of a color component with high sensitivity with respect to the color of the sample solution is used in a case where the sample solution is colored. Accordingly, this method is suitable in a case where the sample solution is colored because the brightness difference $\Delta$ corresponding to a boundary B1 between the already-spread region A1 and the non-spread region A2 can be increased, and the detection accuracy of the brightness difference $\Delta$ can be enhanced.

In addition, in a case where respective signals of the Y signal, the U signal, the V signal, the Cb signal, the Cr signal, the R signal, the G signal, and the B signal are independently used, signal 0 is black and signal 1 is white.

. Although the changes in brightness in the longitudinal direction x may be created on the basis of brightness values of one optional pixel row extending in the longitudinal direction x, the changes are preferably created on the basis of an average value of brightness values for every pixel row extending in the lateral direction y. By using the average value, for example, noise originating from dust adhering to the test piece 1, the cartridge 7, or the like can be reduced.

The brightness change rate illustrated in Fig. 8 is a brightness change rate created from the brightness information of Fig. 7, and shows the brightness change rate of the test piece 1 in the longitudinal direction x. In a case where the dropping section 2 of the test piece 1 is x = 0, brightness values of pixels at positions of x = 0, 1, 2, ..., and 1023 are $Y_0$, $Y_1$, $Y_2$, ..., and $Y_{1023}$, a brightness change rate $Z_n$ at a certain x = n (where n = m + 1, m is an integer of > 1) can be obtained according to, for example, Expression (1).

$$Z_n = Y_n - (Y_{n-m} + Y_{n-m+1} + ... + Y_{n-1})/m \qquad (1)$$

Moreover, the noise can be reduced by obtaining a median value $ZZ_n$ of $Z_n$ to $Z_{n-1}$ (where 1 is an integer of 1 > 1, n > 1) and adopting this $ZZ_n$ as the brightness change rate for determination. In addition, the range of x is changed depending on the pixel resolving power of the imaging unit 12, and is a case where the resolving power in an x direction is 1024 pixels in the above example. Additionally, the value of m can be appropriately set depending on the resolving power and sensitivity of the imaging unit 12.

The brightness difference Δ corresponding to the boundary B appears as a peak P that becomes convex on a positive side in the brightness change rate. For example, in a case where a first threshold value $TV_1$ is set between an experimental value of the brightness change rate (absolute value) of the peak P obtained by actually imaging the test piece 1, onto which the sample solution has been dropped, by the immunological examination apparatus 10, and an experimental value of the brightness change rate (absolute value) between the already-spread region A1 and the non-spread region A2, a brightness difference in which the brightness change rate (absolute value) is equal to or greater than the first threshold value $TV_1$ is detected, and the brightness difference equal to or greater than the first threshold value $TV_1$ is present, the sample solution can be determined to be dropped onto the test piece 1.

An example of setting of the first threshold value $TV_1$ is shown below. First, the Y signal is used for the digitization of the image of the test piece 1, and a brightness average value of 10×10 pixels corresponding to a white portion of the test piece 1 is set to 1. Then, in a case where m and 1 of Expression (1) are set to m = 5 and 1 = 5, respectively, and the brightness change rate $ZZ_n$ of the test piece 1 onto which the sample solution is dropped is obtained, a maximum value (absolute value) of the brightness change rate of the peak P is about 0.035, and a maximum value (absolute value) of the brightness change rate between the already-spread region A1 and the non-spread region A2 is 0.005. In this case, the first threshold value $TV_1$ can be set to (0.035 - 0.005)/2 = 0.015. In addition, the first threshold value $TV_1$ is not necessarily set by the above expression, and can be appropriately set between the brightness change rate of the peak P and the brightness change rate between the already-spread region A1 and the non-spread region A2 in consideration of variations in the brightness change rate between the already-spread region A1 and the non-spread region A2.

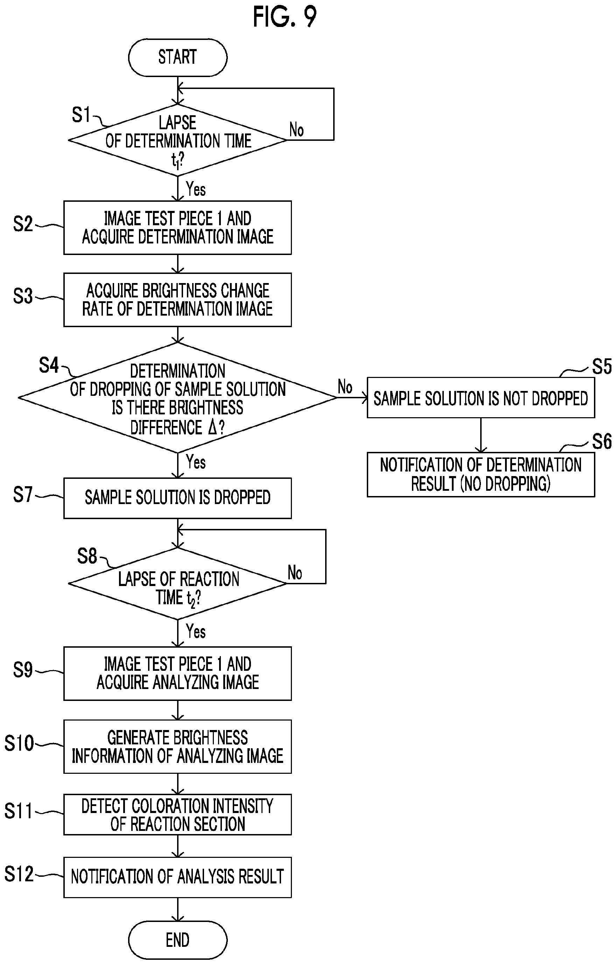

Fig. 9 illustrates a flow of determination processing to be executed by the control unit 15 in a case where whether or not the sample solution is dropped is determined on the basis of the brightness difference.

Assuming that the analysis is started immediately after the sample solution has been dropped onto the test piece 1, in a case where an analysis start instruction is input, the control unit 15 counts the lapse of a predetermined determination time $t_1$ from the start of the analysis (Step S1). The predetermined determination time $t_1$ is a time shorter than the time required for the sample solution dropped onto the test piece 1 to have flowed through the test piece 1, and can be set to, for example, 30 seconds to 1 minute and 30 seconds.

The control unit 15 makes the imaging unit 12 image the test piece 1 after the lapse of the predetermined determination time (Step S2). Then, the control unit 15 acquires the brightness change rate of the determination image in the longitudinal direction x, using the image (determination image) of the test piece 1 acquired in Step S2 (Step S3), and determines whether or not the sample solution is dropped on the basis of the brightness change rate of a determination region set on the determination image (Step S4).

Here, the determination region may be set to the entire region of the determination image, or may be set to a partial region of a determination image including at least a region where the presence of the brightness difference Δ corresponding to the boundary B is predicted when the determination time $t_1$ has lapsed. In a case where the determination region is set to the partial region of the determination image, only the brightness change rate of the determination region may be acquired in the above Step S3. By setting the determination region to the partial region of the determination image, the processing load applied to the control unit 15 can be alleviated.

In determining whether or not the sample solution is dropped, in a case where the brightness difference Δ in which the brightness change rate is equal to or greater than the first threshold value $TV_1$ is not present, the control unit 15 determines that the sample solution is not dropped (Step S5), and notifies the notification unit 13 of the determination result (Step S6). On the other hand, in a case where the brightness difference Δ in which the brightness change rate is equal to or greater than the first threshold value $TV_1$ is present, the control unit 15 determines that the sample solution is dropped (Step S7), and executes analysis processing subsequent to the determination processing.

In the analysis processing subsequent to the determination processing, the control unit 15 counts the lapse of a predetermined reaction time $t_2$ from the start of the analysis (Step S8). The predetermined reaction time $t_2$ is the time during which the reaction section 4 is colored with sufficient intensity for detection in a case where the sample solution including antigens in a predetermined concentration has flowed to the horizontally-placed test piece 1, and can be set to, for example, 20 minutes.

The control unit 15 makes the imaging unit 12 image the test piece 1 after the lapse of the predetermined reaction time (Step S9). Then, the control unit 15 creates brightness information obtained by digitizing the coloration intensity of the reaction section 4 (the determination line 5 and the control line 6), using the image (analyzing image) of the test piece 1 acquired in Step S8 (Step S10).

Then, the control unit 15 detects the coloration intensity of the reaction section 4 (the determination line 5 and the control line 6) on the basis of the brightness information created in Step S10 (Step S11), and notifies the notification unit 13 of the analysis results (the detection result or the quantification result of antigens in the sample solution and the fact that the sample solution has appropriately flowed to the test piece 1) obtained from the detected coloration intensity (Step S12).

In this way, by determining whether or not the sample solution is dropped on the basis of the brightness difference that appears in the determination region of the determination image of the test piece 1, it is possible to early perform notification of the dropping forgetting of the sample solution without waiting for the lapse of the predetermined reaction time $t_2$ at which the coloration of the control line 6 of the reaction section 4 can be detected. It is possible to eliminate the waste in a case where the analysis is performed in the state of the dropping forgetting, and to improve the efficiency of the analysis work. Additionally, since the determination image and the analyzing image of the test piece 1 are acquired by the common imaging unit 12, the size and cost of the immunological examination apparatus 10 can also be reduced.

From a viewpoint of early performing the notification of the dropping forgetting of the sample solution without waiting for lapse of the predetermined reaction time $t_2$ at which the coloration of the control line 6 can be detected, the determination region is preferably set to be on the upstream side of the flow of the sample solution flowing through the test piece 1 with respect to the control line 6 in the image, in a case where the determination region is set to the partial region of the image. As illustrated in Fig. 5, by setting the determination region R to be on the upstream side of the control line 6, the determination time $t_1$ until the leading end of the flow of the sample solution, that is, the boundary B early arrives at the determination region R and thereby, the determination image is acquired from the start of the analysis can be shortened, and the dropping forgetting of the sample solution can be early notified of.

In addition, in the above determination processing, there is a case where the sample solution may flow through the test piece 1 before the predetermined determination time $t_1$ lapses and the boundary B may disappear from the test piece 1. As such a case, a case where the longitudinal direction x of the test piece 1 is inclined with respect to the horizontal due to the installation situation of the immunological examination apparatus 10 and the downstream side of the flow of the sample solution flowing through the test piece 1 is disposed relatively below, and the flow of the sample solution becomes faster is exemplified. On the contrary, in a case where the downstream side of the flow of the sample solution flowing through the test piece 1 is disposed relatively above, there is a possibility that the flow of the sample solution becomes slower and the boundary B is formed in the test piece 1 when the determination time $t_1$ has lapsed. In contrast to these cases, a sensor that detects the inclination of the longitudinal direction x of the test piece 1 with respect to the horizontal may be provided, and the determination time $t_1$ may be changed depending on the inclination detected by the sensor.

Specifically, the determination time $t_1$ may be shortened in the case of an inclination at which the downstream side of the flow of the sample solution flowing through the test piece 1 is disposed relatively above with the determination time $t_1$ in the case where the longitudinal direction x of the test piece 1 is horizontal as a reference, and the determination time $t_1$ may be extended in the case of an inclination at which the downstream side of the flow of the sample solution flowing through the test piece 1 is disposed relatively below. Accordingly, irrespective of the inclination of the longitudinal direction x of the test piece 1 with respect to the horizontal, the boundary B can be put in the image of the test piece 1, and whether or not the sample solution is dropped can be appropriately determined.

However, in a case where the inclination of the longitudinal direction x of the test piece 1 with respect to the horizontal is excessive, that is, in a case where the flow of the sample solution is excessively fast or is excessively slow and the boundary B cannot be put in the image of the test piece 1 even in a case where the determination time $t_1$ is changed depending on the inclination of the longitudinal direction x of the test piece 1 with respect to the horizontal, the above determination method of detecting the brightness difference has is a concern that trouble may occur in the determination of whether or not the sample solution is dropped.

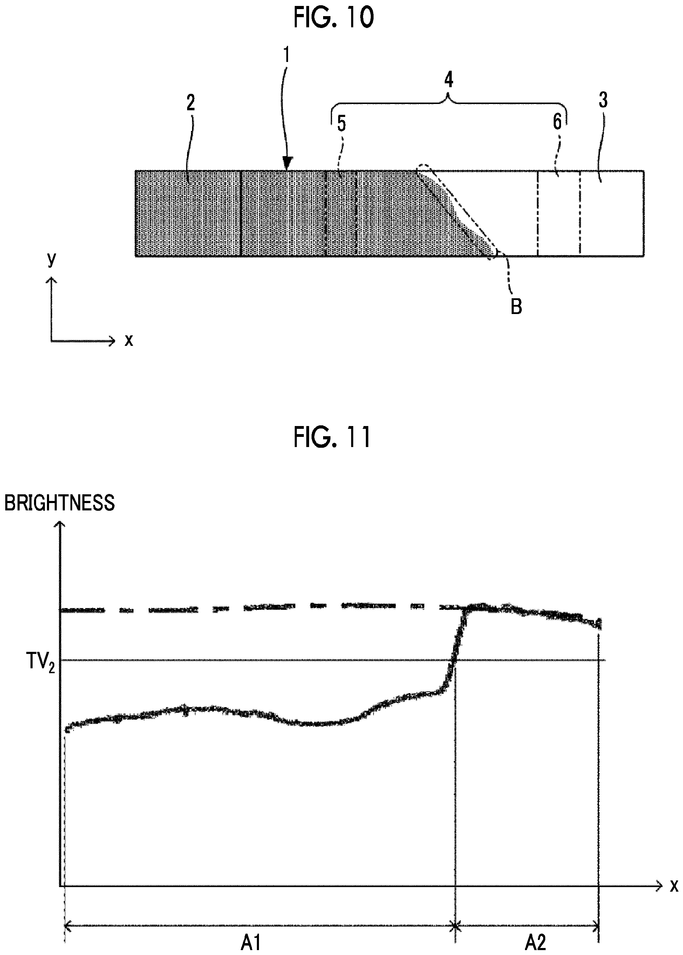

Additionally, in a case where the lateral direction y of the test piece 1 is inclined with respect to the horizontal, and the sample solution has flowed in a biased manner toward one side of the test piece 1 in the lateral direction y, as illustrated in Fig. 10, the boundary B may be inclined with respect to the lateral direction y of the test piece 1. The brightness information used for the detection of the brightness difference, that is, the changes in brightness of the test piece in the longitudinal direction x, are preferably created on the basis of the average value of the brightness values for every pixel row extending in the lateral direction y. However, in a case where the boundary B is inclined with respect to the lateral direction y, there is a possibility that the brightness and the brightness change rate of the brightness difference corresponding to the boundary B may become smaller than an actual brightness and a brightness change rate due to the averaging. In this case, in the above determination method of detecting the brightness difference in which the brightness change rate is equal to or greater than the first threshold value $TV_1$, there is a concern that trouble may occur in the determination of whether or not the sample solution is dropped.

In a determination method to be described below, dark pixels of which the brightness values are equal to or smaller than a second threshold value in the image of the test piece 1 are detected, and whether or not the sample solution is dropped is determined on the basis of the number of dark pixels.

Fig. 11 illustrates the brightness information obtained by digitizing the image of the test piece 1 depending on the brightness, and illustrates brightness values of respective pixels included in one pixel row extending in the longitudinal direction x of the test piece 1. In the drawing, a solid line represents brightness information in a case the sample solution is dropped, and a one-dot chain line represents brightness information in a case where the sample solution is not dropped.

The test piece 1 in the case where the sample solution is not dropped is white, and the brightness values of the respective pixels to be shown depending on the brightness information are generally high values. On the other hand, in the test piece 1 in the case where the sample solution is dropped, the already-spread region A1 of which the color tone is relatively dark becomes wide as the sample solution flows slightly, and brightness values of pixels corresponding to the already-spread region A1 out of the brightness values of the respective pixels shown depending on the brightness information are relatively low values.

A second threshold value $TV_2$ is set between an experimental value of a brightness value of the test piece 1 in a case the sample solution is not dropped and an experimental value of a brightness value of the already-spread region A1, and in a case where dark pixels of which the brightness values are equal to or smaller than the second threshold value $TV_2$, and the pixel number of the dark pixels is equal to or greater than a predetermined number are detected, it can be determined that the already-spread region A1 is present, that is, the sample solution is dropped onto the test piece 1.

An example of setting of the second threshold value $TV_2$ is shown below. The brightness of a pixel at a position of x = n and y = p is set to Y (n, p), a median value of Y(n-q, p-q) to Y(n+q, p+q), that is, a median value of brightnesss in a region of 2q x 2q centered on coordinates (n, p) is set to YY(n, p), and a maximum value of the brightnesss is set to 1. In a case where a minimum value of YY(n, p) of the test piece 1 in which the sample solution is not dropped is 0.94 and a maximum value of YY(n, p) of the already-spread region A1 of the test piece 1 in which the sample solution is dropped is 0.72, the second threshold value $TV_2$ can be set to (0.94 + 0.72)/2 = 0.83. In addition, the second threshold value $TV_2$ is not necessarily set according to the above expression, and can be appropriately set between the minimum value of YY(n, p) in the case where the sample solution is not dropped and the maximum value of YY(n, p) in a case where the sample solution is dropped, and it is possible to enhance the detection accuracy of dark pixels in a case where the second threshold value $TV_2$ is set to a larger value (for example, 0.88) than 0.83.

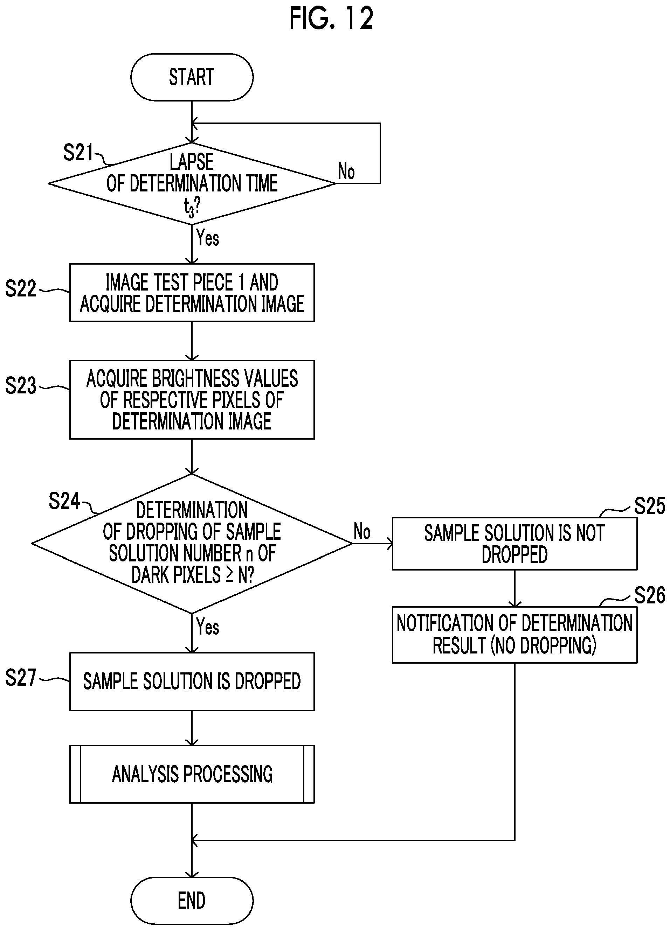

Fig. 12 illustrates a flow of determination processing to be executed by the control unit 15 in a case where whether or not the sample solution is dropped is determined on the basis of the pixel number of dark pixels.

In a case where an analysis start instruction is input, the control unit 15 counts the lapse of a predetermined determination time $t_3$ from the start of the analysis (Step S21). The predetermined determination time $t_3$ may be the same as or different from the determination time $t_1$ used for the above determination processing of detecting the brightness difference.

The control unit 15 makes the imaging unit 12 image the test piece 1 after the lapse of the predetermined determination time (Step S22). The control unit 15 acquires the brightness values of the respective pixels of this determination image, using the image (determination image) of the test piece 1 acquired in Step S2 (Step S23).

Then, the control unit 15 applies the second threshold value $TV_2$ to the brightness values of the respective pixels of the determination image acquired in Step S23, thereby detecting dark pixels within the determination region set in the determination image, and determines whether or not the sample solution is dropped on the basis of the pixel number n of the detected dark pixels (Step S24). The determination region may be set to the entire region of the determination image, may be set to the partial region of the determination image, and is preferably set to the upstream side of the flow of the sample solution flowing through the test piece 1 with respect to than the control line 6 in the image in a case where the determination region is set to the partial region of the determination image.

In the determination of whether or not the sample solution is dropped, in a case where the pixel number n of the dark pixels is smaller than the predetermined number N, the control unit 15 determines that the sample solution is not dropped (Step S25), and notifies the notification unit 13 of the determination result (Step S26). On the other hand, in a case where the pixel number n of the dark pixels is equal to or greater than the predetermined number N, the control unit 15 determines that the sample solution is dropped (Step S27), and executes analysis processing subsequent to the determination processing.

The predetermined number N is not particularly limited and may be, for example, N = 1, but the predetermined number N is preferably plural. There is a possibility that the noise resulting from the dust adhering to the test piece 1, the cartridge 7, or the like is detected as dark pixels, and there is also a possibility that the illumination unevenness on the test piece 1 resulting from the setting of the light source 21 of the imaging unit 12 may be detected as dark pixels. However, by setting the predetermined number N to a plural number, influence of the noise, the illumination unevenness, or the like can be reduced, and the determination accuracy can be enhanced.

Since the analysis processing subsequent to the determination processing is the same as that of Step S8 to Step S12 illustrated in Fig. 9, the description thereof is omitted.

In this way, by determining whether or not the sample solution is dropped on the basis of the pixel number of the dark pixels included in the determination region of the determination image of the test piece 1, it is possible to early perform notification of the dropping forgetting of the sample solution without waiting for the lapse of the predetermined reaction time $t_2$ at which the coloration of the control line 6 of the reaction section 4 can be detected. Also, it is possible to eliminate the waste in a case where the analysis is performed in the state of the dropping forgetting, and to improve the efficiency of the analysis work. Moreover, since the dark pixels are generated in the determination image of the test piece 1 as the sample solution flows even slightly through the test piece 1, and are generated in the determination image of the test piece 1 even in a case where the sample solution has flowed through the test piece 1, whether or not the sample solution is dropped can be stably determined irrespective of the inclination of the longitudinal direction x of the test piece 1 with respect to the horizontal.

In addition, whether or not the sample solution is dropped can be determined also by detecting bright pixels.

Referring back to Fig. 11, out of the brightness values of the respective pixels shown depending on the brightness information of the test piece 1 in a case where the sample solution is dropped, the brightness values of the pixels corresponding to the already-spread region A1 are the relatively low values, and the brightness values of the pixels corresponding to the non-spread region A2 are the relatively high values. Then, in a case where the sample solution flows with the lapse of time, the already-spread region A1 is increased and conversely, the non-spread region A2 is reduced.

In a case where a third threshold value $TV_3$ is set between the experimental value of the brightness value of the already-spread region A1 and an experimental value of a brightness value of the non-spread region A2, bright pixels of which the brightness values are equal to or greater than the third threshold value $TV_3$ for each of a plurality of the determination images acquired at appropriate time intervals are detected, and the pixel number of the bright pixels of each of the plurality of determination images decreases in order of acquisition of the images, it can be determined that the non-spread region A2 decreases, that is, the sample solution is dropped onto the test piece 1.

An example of setting of the third threshold value $TV_3$ is shown below. The brightness of a pixel at a position of x = n and y = p is set to Y (n, p), a median value of Y(n-q, p-q) to Y(n+q, p+q), that is, a median value of brightnesss in a region of 2q x 2q centered on coordinates (n, p) is set to YY(n, p), and a maximum value of the brightnesss is set to 1. In a case where a minimum value of YY(n, p) of the test piece 1 in which the sample solution is not dropped, that is, the non-spread region A2 is 0.94 and a maximum value of YY(n, p) of the already-spread region A1 of the test piece 1 in which the sample solution is dropped is 0.72, the third threshold value $TV_3$ can be set to (0.94 + 0.72)/2 = 0.83. In addition, the third threshold value $TV_3$ is not necessarily set according to the above expression, and can be appropriately set between the minimum value of YY(n, p) in the case where the sample solution is not dropped and the maximum value of YY(n, p) in a case where the sample solution is dropped, and it is possible to enhance the detection accuracy of bright pixels in a case where the third threshold value $TV_3$ is set to a smaller value (for example, 0.78) than 0.83.

Then, in a case where j images (where j is an integer of > 1) are acquired and $a_{j-1} - a_j > \alpha$ ($\alpha$ is an integer of > 0) is always established with the number of bright pixels of each image as aj, it can be determined that the pixel number of the bright pixels decreases in order of acquisition of the images. Preferably, j is three or more, and $\alpha$ can be appropriately set on the basis of noise including defective pixels of the imaging unit 12. For example, j = 5 and $\alpha$ = 10 can be established.

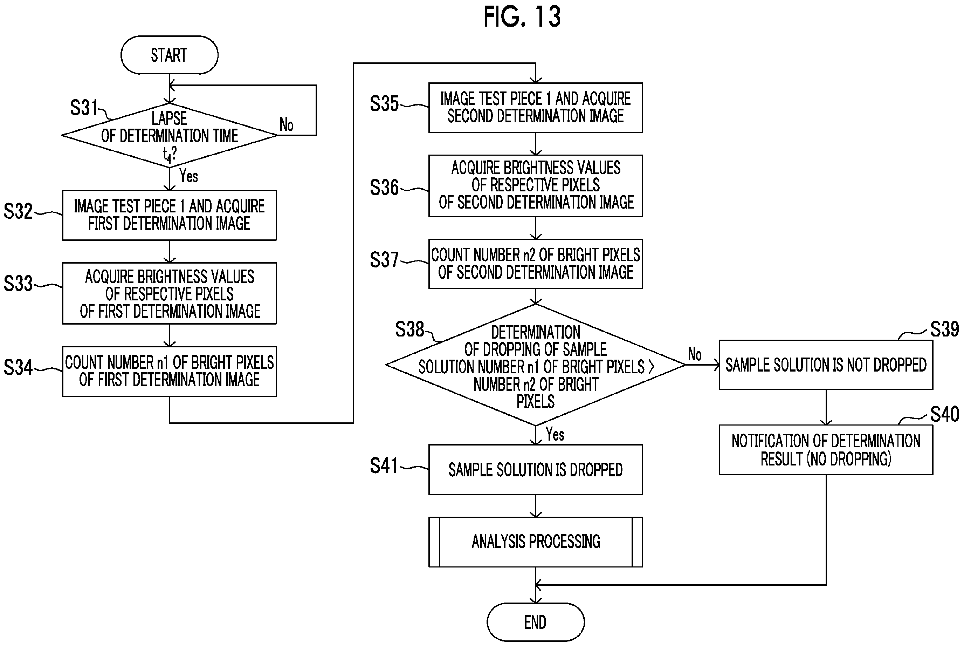

Fig. 13 illustrates a flow of determination processing to be executed by the control unit 15 in a case where whether or not the sample solution is dropped is determined on the basis of the pixel number of bright pixels. In addition, although a case where two determination images are acquired will be described below, the determination images are not limited to two, and may be three or more.

In a case where an analysis start instruction is input, the control unit 15 counts the lapse of a predetermined determination time $t_4$ from the start of the analysis (Step S31). The predetermined determination time $t_4$ may be the same as or different from the determination time $t_1$ used for the above determination processing of detecting the brightness difference.

The control unit 15 makes the imaging unit 12 image the test piece 1 after the lapse of the predetermined determination time (Step S32). Then, the control unit 15 acquires the brightness values of the respective pixels of the first determination image, using the image (first determination image) of the test piece 1 acquired in Step S32 (Step S33).

Then, the control unit 15 applies the third threshold value $TV_3$ to the brightness values of the respective pixels of the first determination image acquired in Step S33, thereby detecting bright pixels within the determination region set in the first determination image, and counts the pixel number n1 of the detected bright pixels (Step S34). The determination region may be set the entire region of the determination image, may be set to the partial region of the determination image, and is preferably set to the upstream side of the flow of the sample solution flowing through the test piece 1 with respect to than the control line 6 in the image in a case where the determination region is set to the partial region of the determination image.

Additionally, after the control unit 15 makes the imaging unit 12 image the test piece 1 in Step S32, the control unit 15 makes the imaging unit 12 image the test piece 1 again at appropriate time intervals (for example, 5 seconds) (Step S35). The control unit 15 acquires the brightness values of the respective pixels of a second determination image, using the image (second determination image) of the test piece 1 acquired in Step S35 (Step S36).

Then, the control unit 15 applies the third threshold value $TV_3$ to the brightness values of the respective pixels of the second determination image acquired in Step S36, thereby detecting bright pixels within the determination region set in the second determination image, and counts the pixel number n2 of the detected bright pixels (Step S37). In addition, the determination region set in a second determination image is the same as the determination region set in the first determination image.

The control unit 15 determines whether or not the sample solution is dropped on the basis of the pixel number $n_1$ of the bright pixels of the first determination image counted in Step S34 and the pixel number $n_2$ of the bright pixels of the second determination image counted in Step S37 (Step S38).

In the determination of whether or not the sample solution is dropped, in a case where the pixel number $n_2$ of the bright pixels of the second determination image is equal to or greater than the pixel number $n_1$ of the bright pixels of the first determination image, that is, does not decrease, the control unit 15 determines that the sample solution is not dropped (Step S39), and notifies the notification unit 13 of the determination result (Step S40). On the other hand, in a case where the pixel number $n_2$ of the bright pixels of the second determination image is smaller than the pixel number $n_1$ of the bright pixels of the first determination image, that is, decreases, the control unit 15 determines that the sample solution is dropped (Step S41), and executes analysis processing subsequent to the determination processing.

Since the analysis processing subsequent to the determination processing is the same as that of Step S8 to Step S12 illustrated in Fig. 9, the description thereof is omitted.

In this way, by determining whether or not the sample solution is dropped on the basis of the pixel number of the bright pixels included in the determination region of the determination image of the test piece 1, it is possible to early perform notification of the dropping forgetting of the sample solution without waiting for the lapse of the predetermined reaction time $t_2$ at which the coloration of the control line 6 of the reaction section 4 can be detected. Also, it is possible to eliminate the waste in a case where the analysis is performed in the state of the dropping forgetting, and to improve the efficiency of the analysis work. Moreover, since reduction in the pixel number of bright pixels between a plurality of determination images is not influenced by the noise, the illumination unevenness, or the like that may be detected as dark pixels, whether or not the sample solution is dropped can be stably determined.

Although the respective determination methods of the determination method of detecting the brightness difference, the determination method of detecting the dark pixels, and the determination method of detecting the bright pixels have been individually described up to now, a plurality of determination methods may be combined together.

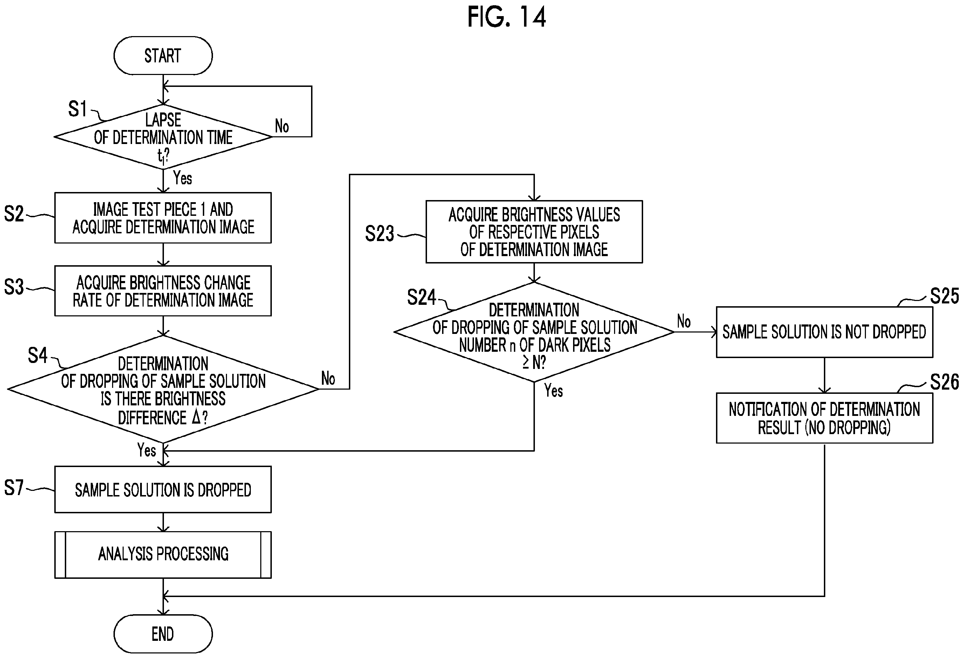

An example illustrated in Fig. 14 is obtained by combining the determination method of detecting the brightness difference illustrated in Fig. 9 with the determination method of detecting the dark pixels illustrated in Fig. 12. The control unit 15 determines whether or not the sample solution is dropped on the basis of the brightness change rate of the determination image (Step S4). In a case where the brightness difference $\Delta$ in which the brightness change rate is equal to or greater than the first threshold value $TV_1$ is not present in the determination image (Step S4-No), the control unit 15 further detects the dark pixels of the determination image to determine whether or not the sample solution is dropped on the basis of the pixel number n of the dark pixels (Step S24). Then, in a case where the pixel number n of the dark pixels is smaller than the predetermined number N, the control unit 15 determines that the sample solution is not dropped (Step S25). By combining the plurality of determination methods in this way, the determination accuracy in a case where it is determined the sample solution is not dropped can be enhanced.

In the example illustrated in Fig. 14, the determination method of detecting the brightness difference and the determination method of detecting the dark pixels are combined together. However, the determination method of detecting the brightness difference and the determination method of detecting the bright pixels may be combined together, the determination method of detecting the dark pixels and the determination method of detecting the bright pixels may be combined together, and the determination method of detecting the brightness difference, the determination method of detecting the dark pixels, and the determination method of detecting the bright pixels may be combined together.

As described above, the immunological examination apparatus disclosed in the present specification is an immunological examination apparatus that images a chromatographic test piece onto which a sample solution is dropped and through which the dropped sample solution is made to flow in a longitudinal direction and that analyzes the sample solution on the basis of the obtained image. The immunological examination apparatus comprises an imaging unit that images the chromatographic test piece, a determination unit that determines presence or absence of the dropping of the sample solution onto the chromatographic test piece on the basis of an image acquired by the imaging unit, and a notification unit that gives a notification of a determination result of the determination unit. The determination unit acquires a brightness change rate of the chromatographic test piece in the longitudinal direction in a determination region set in the image, and determines that the sample solution has been dropped in a case where a brightness difference in which an absolute value of the brightness change rate is equal to or greater than a first threshold value is present.

Additionally, the immunological examination apparatus disclosed in the present specification is an immunological examination apparatus that images a chromatographic test piece onto which a sample solution is dropped and through which the dropped sample solution is made to flow in a longitudinal direction and that analyzes the sample solution on the basis of the obtained image. The immunological examination apparatus comprises an imaging unit that images the chromatographic test piece, a determination unit that determines presence or absence of the dropping of the sample solution onto the chromatographic test piece on the basis of an image acquired by the imaging unit, and a notification unit that gives a notification of a determination result of the determination unit. The determination unit detects dark pixels of which brightness values are equal to or smaller than a second threshold value, in a determination region set in the image, and determines that the sample solution has been dropped in a case where a predetermined number or more of the dark pixels are present.

Additionally, the immunological examination apparatus disclosed in the present specification is an immunological examination apparatus that images a chromatographic test piece onto which a sample solution is dropped and through which the dropped sample solution is made to flow in a longitudinal direction and that analyzes the sample solution on the basis of the obtained image. The immunological examination apparatus comprises an imaging unit that images the chromatographic test piece, a determination unit that determines presence or absence of the dropping of the sample solution onto the chromatographic test piece on the basis of an image acquired by the imaging unit, and a notification unit that gives a notification of a determination result of the determination unit. The determination unit detects bright pixels of which brightness values are equal to or greater than a third threshold value for each of a plurality of images in a determination region set in the image, and determines that the sample solution has been dropped in a case where the pixel number of the bright pixels of each of the images decreases in order of acquisition of the images.

Additionally, the chromatographic test piece has a reaction section that is colored due to contact with the sample solution, and the determination region is set to an upstream side of a flow of the sample solution flowing through the chromatographic test piece with respect to the reaction section in the image.

Additionally, the determination unit determines the presence or absence of the dropping of the sample solution onto the chromatographic test piece on the basis of the image acquired by the imaging unit at a timing before an acquisition timing of an analyzing image used for the analysis of the sample solution.

Additionally, the imaging unit acquires the analyzing image.

The invention can provide the immunological examination apparatus capable of giving an early notification of dropping forgetting of the sample solution onto the chromatographic test piece.

Although the embodiment of the invention has been described in detail above, this is merely an example, and the invention can be carried out in aspects to which various changes are added, without departing from the scope of the scope. The present application is based on Japanese Patent application (JP2017-025362) filed on February 14, 2017, the contents of which are incorporated herein by reference.

Explanation of References

1: chromatographic test piece
2: dropping section
3: spreading section
4: reaction section
5: determination line
6: control line
7: cartridge
10: immunological examination apparatus
11: operating unit
12: imaging unit
13: notification unit
14: storage unit
15: control unit
20: installation unit
21: light source
22: imaging element
23: image processing unit
24: analysis unit
25: determination unit
a: antigen
b: labeled antibody
ab: antigen-antibody complex
c: first capture antibody

d: second capture antibody
A1: already-spread region
A2: non-spread region
B: boundary
n: pixel number
N: predetermined number
n1: pixel number
n2: pixel number
P: peak
R: determination region
$t_1$: determination time
$t_2$: reaction time
$t_3$: determination time
$t_4$: determination time
$TV_1$: first threshold value
$TV_2$: second threshold value
$TV_3$: third threshold value
x: longitudinal direction
Y: lateral direction
$\Delta$: brightness difference

**Claims**

1. An immunological examination apparatus that images a chromatographic test piece onto which a sample solution is dropped and on which the sample solution dropped is made to flow in a longitudinal direction and that analyzes the sample solution on the basis of an acquired image, the immunological examination apparatus comprising:

   an imaging unit that images the chromatographic test piece;
   a determination unit that determines presence or absence of a dropping of the sample solution onto the chromatographic test piece on the basis of an image acquired by the imaging unit; and
   a notification unit that gives a notification of a determination result of the determination unit,
   wherein the determination unit

      acquires a brightness change rate of the chromatographic test piece in the longitudinal direction in a determination region set in the image, and
      determines that the sample solution has been dropped in a case where a brightness difference in which an absolute value of the brightness change rate is equal to or greater than a first threshold value is present.

2. An immunological examination apparatus that images a chromatographic test piece onto which a sample solution is dropped and on which the sample solution dropped is made to flow in a longitudinal direction and that analyzes the sample solution on the basis of an acquired image, the immunological examination apparatus comprising:

   an imaging unit that images the chromatographic test piece;
   a determination unit that determines presence or absence of a dropping of the sample solution onto the chromatographic test piece on the basis of an image acquired by the imaging unit; and
   a notification unit that gives a notification of a determination result of the determination unit,
   wherein the determination unit

      detects a dark pixel of which a brightness value is equal to or smaller than a second threshold value, in a determination region set in the image, and
      determines that the sample solution has been dropped in a case where a predetermined number or more of the dark pixel is present.

3. An immunological examination apparatus that images a chromatographic test piece onto which a sample solution is dropped and on which the sample solution dropped is made to flow in a longitudinal direction and that analyzes the sample solution on the basis of an acquired image, the immunological examination apparatus comprising:

an imaging unit that images the chromatographic test piece;
a determination unit that determines presence or absence of a dropping of the sample solution onto the chromatographic test piece on the basis of an image acquired by the imaging unit; and
a notification unit that gives a notification of a determination result of the determination unit,
wherein the determination unit

detects a bright pixel of which a brightness value is equal to or greater than a third threshold value for each of a plurality of the images in a determination region set in the image, and
determines that the sample solution has been dropped in a case where a pixel number of the bright pixel of each of the images decreases in order of acquisition of the images.

4. The immunological examination apparatus according to any one of claims 1 to 3, wherein the chromatographic test piece has a reaction section that is colored due to contact with the sample solution, and
wherein the determination region is set at an upstream side of a flow of the sample solution flowing on the chromatographic test piece with respect to the reaction section in the image.

5. The immunological examination apparatus according to any one of claims 1 to 4, wherein the determination unit determines the presence or absence of the dropping of the sample solution onto the chromatographic test piece on the basis of the image acquired by the imaging unit at a timing before an acquisition timing of an analyzing image used in analyzing the sample solution.

6. The immunological examination apparatus according to claim 5,
wherein the imaging unit acquires the analyzing image.

FIG. 1

FIG. 3

# FIG. 3

10

ANALYSIS APPARATUS

11 — OPERATING UNIT

12 — IMAGING UNIT
21 — LIGHT SOURCE
22 — IMAGING ELEMENT

CONTROL UNIT

IMAGE PROCESSING UNIT — 23

ANALYSIS UNIT — 24

DETERMINATION UNIT — 25

15

NOTIFICATION UNIT — 13

STORAGE UNIT — 14

# FIG. 4

# FIG. 5

# FIG. 6

# FIG. 7

## FIG. 8

# FIG. 9

START

S1 — LAPSE OF DETERMINATION TIME $t_1$? — No

Yes

S2 — IMAGE TEST PIECE 1 AND ACQUIRE DETERMINATION IMAGE

S3 — ACQUIRE BRIGHTNESS CHANGE RATE OF DETERMINATION IMAGE

S4 — DETERMINATION OF DROPPING OF SAMPLE SOLUTION IS THERE BRIGHTNESS DIFFERENCE Δ? — No — S5 — SAMPLE SOLUTION IS NOT DROPPED

S6 — NOTIFICATION OF DETERMINATION RESULT (NO DROPPING)

Yes

S7 — SAMPLE SOLUTION IS DROPPED

S8 — LAPSE OF REACTION TIME $t_2$? — No

Yes

S9 — IMAGE TEST PIECE 1 AND ACQUIRE ANALYZING IMAGE

S10 — GENERATE BRIGHTNESS INFORMATION OF ANALYZING IMAGE

S11 — DETECT COLORATION INTENSITY OF REACTION SECTION

S12 — NOTIFICATION OF ANALYSIS RESULT

END

# FIG. 10

# FIG. 11

# FIG. 12

```
                    ┌──────────┐
                    │  START   │
                    └────┬─────┘
                         │
                         ▼
S21 ──────◇ LAPSE OF DETERMINATION TIME t₃? ◇──── No ──┐
              │ Yes                                     │
              ▼                                         │
S22 ── IMAGE TEST PIECE 1 AND                           │
       ACQUIRE DETERMINATION IMAGE                      │
              │                                         │
              ▼                                         │
S23 ── ACQUIRE BRIGHTNESS VALUES                        │
       OF RESPECTIVE PIXELS OF                          │
       DETERMINATION IMAGE                              │
              │                                         │
              ▼                                         │
S24 ◇ DETERMINATION OF DROPPING OF SAMPLE ◇── No ── S25 SAMPLE SOLUTION IS NOT DROPPED
     SOLUTION NUMBER n OF DARK PIXELS ≥ N?                          │
              │ Yes                                                 ▼
              ▼                                     S26 NOTIFICATION OF DETERMINATION
S27 ── SAMPLE SOLUTION IS DROPPED                       RESULT (NO DROPPING)
              │
              ▼
       ANALYSIS PROCESSING
              │
              ▼
        ┌──────────┐
        │   END    │
        └──────────┘
```

S21 — LAPSE OF DETERMINATION TIME $t_3$? — No

Yes

S22 — IMAGE TEST PIECE 1 AND ACQUIRE DETERMINATION IMAGE

S23 — ACQUIRE BRIGHTNESS VALUES OF RESPECTIVE PIXELS OF DETERMINATION IMAGE

S24 — DETERMINATION OF DROPPING OF SAMPLE SOLUTION NUMBER $n$ OF DARK PIXELS $\geq N$? — No

S25 — SAMPLE SOLUTION IS NOT DROPPED

S26 — NOTIFICATION OF DETERMINATION RESULT (NO DROPPING)

Yes

S27 — SAMPLE SOLUTION IS DROPPED

ANALYSIS PROCESSING

END

# FIG. 13

START

S31 — LAPSE OF DETERMINATION TIME $t_4$? — No

Yes

S32 — IMAGE TEST PIECE 1 AND ACQUIRE FIRST DETERMINATION IMAGE

S33 — ACQUIRE BRIGHTNESS VALUES OF RESPECTIVE PIXELS OF FIRST DETERMINATION IMAGE

S34 — COUNT NUMBER $n1$ OF BRIGHT PIXELS OF FIRST DETERMINATION IMAGE

S35 — IMAGE TEST PIECE 1 AND ACQUIRE SECOND DETERMINATION IMAGE

S36 — ACQUIRE BRIGHTNESS VALUES OF RESPECTIVE PIXELS OF SECOND DETERMINATION IMAGE

S37 — COUNT NUMBER $n2$ OF BRIGHT PIXELS OF SECOND DETERMINATION IMAGE

S38 — DETERMINATION OF DROPPING OF SAMPLE SOLUTION NUMBER $n1$ OF BRIGHT PIXELS > NUMBER $n2$ OF BRIGHT PIXELS — No — S39 SAMPLE SOLUTION IS NOT DROPPED

Yes

S41 — SAMPLE SOLUTION IS DROPPED

ANALYSIS PROCESSING

S40 — NOTIFICATION OF DETERMINATION RESULT (NO DROPPING)

END

EP 3 584 577 A1

# FIG. 14

```
                    ┌──────────┐
                    │  START   │
                    └──────────┘
                         │
                         ▼
         ┌─────────────────────────────┐ No
    S1 ──│   LAPSE OF DETERMINATION     │────┐
         │         TIME tᵢ?             │    │
         └─────────────────────────────┘    │
                    │ Yes                    │
                    ▼                        │
         ┌─────────────────────────┐         │     ┌──────────────────────────┐
    S2 ──│  IMAGE TEST PIECE 1 AND │         │ S23─│  ACQUIRE BRIGHTNESS VALUES│
         │ ACQUIRE DETERMINATION   │         │     │  OF RESPECTIVE PIXELS     │
         │        IMAGE            │         │     │  OF DETERMINATION IMAGE   │
         └─────────────────────────┘         │     └──────────────────────────┘
                    │                         │               │
                    ▼                         │               ▼
         ┌─────────────────────────┐          │   ┌──────────────────────────┐
    S3 ──│  ACQUIRE BRIGHTNESS      │         │   S24│  DETERMINATION OF        │ No   ┌─────────────────────────┐
         │  CHANGE RATE OF          │         │     │  DROPPING OF SAMPLE       │─────▶│ SAMPLE SOLUTION IS       │── S25
         │  DETERMINATION IMAGE     │         │     │  SOLUTION NUMBER n OF     │      │ NOT DROPPED             │
         └─────────────────────────┘          │     │  DARK PIXELS ≥ N?        │      └─────────────────────────┘
                    │                         │     └──────────────────────────┘                │
                    ▼                         │               │ Yes                             ▼
    S4 ── DETERMINATION OF DROPPING OF        │               │              ┌─────────────────────────┐
          SAMPLE SOLUTION IS THERE    No      │               │              │ NOTIFICATION OF         │── S26
          BRIGHTNESS DIFFERENCE Δ? ───────────┘               │              │ DETERMINATION RESULT    │
                    │ Yes                                      │              │ (NO DROPPING)           │
                    ◀─────────────────────────────────────────┘              └─────────────────────────┘
                    ▼                                                                     │
         ┌─────────────────────────┐                                                     │
    S7 ──│ SAMPLE SOLUTION IS       │                                                     │
         │ DROPPED                  │                                                     │
         └─────────────────────────┘                                                     │
                    │                                                                     │
                    ▼                                                                     │
         ┌─────────────────────────┐                                                     │
         │   ANALYSIS PROCESSING    │                                                     │
         └─────────────────────────┘                                                     │
                    │                                                                     │
                    ◀─────────────────────────────────────────────────────────────────────┘
                    ▼
              ┌──────────┐
              │   END    │
              └──────────┘
```

## INTERNATIONAL SEARCH REPORT

| | International application No. |
|---|---|
| | PCT/JP2018/001060 |

| A. CLASSIFICATION OF SUBJECT MATTER |
|---|
| Int.Cl. G01N33/543(2006.01)i, G01N33/483(2006.01)i |

According to International Patent Classification (IPC) or to both national classification and IPC

| B. FIELDS SEARCHED |
|---|
| Minimum documentation searched (classification system followed by classification symbols) |
| Int.Cl. G01N33/543, G01N33/483 |

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| X/Y | JP 2010-101758 A (PANASONIC CORPORATION) 06 May 2010, entire text, all drawings, particularly, claims, paragraphs [0010]-[0012], [0032], [0034], [0041], fig. 1, 5 (Family: none) | 1, 4-6/2-3 |
| X/Y | JP 2010-032447 A (PANASONIC CORPORATION) 12 February 2010, entire text, all drawings, particularly, paragraph [0053], fig. 4, 5, etc. (Family: none) | 1/2-3 |

☒ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11.04.2018 | 24.04.2018 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2018/001060

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 09-288730 A (DAM SUIGENCHI KANKYO SEIBI CENTER) 04 November 1997, entire text, all drawings, etc. (Family: none) | 2-3 |
| A | JP 2013-174519 A (FUJIFILM CORPORATION) 05 September 2013, entire text, all drawings (Family: none) | 1-6 |
| A | WO 2009/128205 A1 (PANASONIC CORPORATION) 22 October 2009, entire text, all drawings, etc. & US 2011/0032525 A1 & EP 2270421 A1 | 1-6 |

Form PCT/ISA/210 (continuation of second sheet) (January 2015)

**EP 3 584 577 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010032447 A
- JP 2017025362 A